Europäisches Patentamt

European Patent Office

Office européen des brevets

(11) Numéro de publication : **0 475 818 A1**

# (12) DEMANDE DE BREVET EUROPEEN

(21) Numéro de dépôt : **91402356.9**

(51) Int. Cl.⁵ : **G06F 15/70**

(22) Date de dépôt : **03.09.91**

(30) Priorité : **06.09.90 FR 9011086**

(43) Date de publication de la demande :
**18.03.92 Bulletin 92/12**

(84) Etats contractants désignés :
**DE NL**

(71) Demandeur : **GENERAL ELECTRIC CGR S.A.**
**100, rue Camille-Desmoulins**
**F-92130 Issy les Moulineaux (FR)**

(72) Inventeur : **Lienard, Jean**
**Cabinet Ballot-Schmit, 7, rue Le Sueur**
**F-75116 Paris (FR)**
Inventeur : **Leclerc, Vincent**
**Cabinet Ballot-Schmit, 7, rue Le Sueur**
**F-75116 Paris (FR)**

(74) Mandataire : **Schmit, Christian Norbert Marie**
**et al**
**Cabinet Ballot-Schmit 7, rue Le Sueur**
**F-75116 Paris (FR)**

(54) **Procédé de détermination des caractéristiques d'une structure vasculaire.**

(57) Pour déterminer les caractéristiques, notamment les dimensions, d'une sténose vasculaire, dans le but de la soumettre à une angioplastie transluminale, on agrandit dans un écran de visualisation l'image de cette sténose. On se sert des bords d'écran comme représentatifs d'un tracé du contour de l'image. A l'intérieur de ce périmètre fermé on recherche les bords des vaisseaux, leurs axes moyens, et leurs sections d'une manière automatique. On affiche la variation entre le maximum et le minimum de cette section, c'est-à-dire le rétrécissement correspondant de la sténose. On montre que cette manière d'agir nécessite peu d'ordres de la part du praticien qui l'effectue et qu'en particulier ce nombre peu important d'ordres est compatible avec une commande de type vocale. Dans ces conditions le praticien peut travailler à mains libres sur le patient sans être préoccupé des manipulations propres à la mesure des caractéristiques.

EP 0 475 818 A1

FIG_1

La présente invention a pour objet un procédé de détermination des caractéristiques d'une sténose vasculaire. Elle est utilisable dans le domaine de la radiologie médicale. Elle a essentiellement pour but d'aboutir à une meilleure ergonomie pour le praticien qui est en train d'intervenir sur un patient. L'intervention dont il est question est dans la plupart des cas une angioplastie transluminale.

Une telle opération est une opération intravasculaire au cours de laquelle un praticien dilate un vaisseau sténosé à l'aide d'un ballonnet gonflable porté par l'extrémité d'un cathéter que ce praticien a introduit dans le système vasculaire. La sténose représentant un athérome, qui limite l'écoulement du sang, le fait de dilater le vaisseau à l'endroit de la sténose redonne à ce vaisseau une section normale, et lui permet d'assurer un débit de sang normal. Les rétrécissements qui sont ainsi traités sont en général des rétrécissements dus à l'adhésion d'une plaque d'athérome sur les parois du vaisseau. Le choix du ballonnet gonflable, et de la pression de gonflage à laquelle il sera soumis, est dicté par la nature relative du rétrécissement et par une calibration de la section du vaisseau. Pour que l'angioplastie soit efficace, il faut que la dilatation du vaisseau soit exercée au delà de la limite d'élasticité de ce vaisseau, de telle façon que la sténose ne puisse pas reprendre, quelques mois plus tard, une allure aussi néfaste. Cependant, il importe de ne pas exagérer cette dilatation. Le choix des dimensions du ballonnet est donc primordial.

Jusqu'à dernièrement, le choix de ce ballonnet s'effectuait empiriquement, sur des critères et des connaissances anatomiques du praticien pratiquant l'intervention. Avec la diffusion des systèmes d'imagerie radiologique numérisée, ce choix peut aujourd'hui être conforté par une mesure préalable de la taille du vaisseau sain proche de la sténose et par une surveillance en temps réel de l'intervention.

La plupart des systèmes d'angiographie numérisée commercialisés aujourd'hui sont équipés de cette fonctionnalité de mesure. Mais celle-ci implique encore de la part du praticien une interaction importante avec le système, notamment au moyen d'un clavier, d'une boule traçante, de touches, de potentiomètres, ou de crayons lumineux. Le praticien est donc confronté à un problème d'intervention médicale sur un patient, (pour introduire le cathéter et surveiller sa progression sur un écran) simultanément à la manipulation d'un système numérique de mesure sur image.

Le principe des mesures connu à ce jour consiste
– à faire déplacer à un opérateur, le praticien en pratique, des index lumineux de part et d'autre de la sténose pour délimiter un champ d'étude ;
– à lui faire déplacer deux autres index pour mesurer au droit de ces autres index la section du vaisseau sanguin ;
– un index étant placé à l'endroit de la sténose et un autre à l'endroit d'une partie saine du vaisseau, et
– à faire le calcul automatique des variations relatives des dimensions du vaisseau au passage de la sténose.

Bien que simple dans son principe, ce système présente l'inconvénient que le praticien doit agir sur les boutons du clavier pour
– choisir un premier index sur un clavier,
– agir sur une boule ou une souris pour déplacer cet index d'un côté de la sténose ;
– choisir un autre index et à nouveau agir sur la boule pour déplacer cet autre index de l'autre côté de la sténose ;
– éventuellement provoquer un agrandissement, un zoom, de manière à y voir mieux. Une fois que le champ a été ainsi délimité, il lance une opération de détection automatique des contours du tronçon de vaisseau délimité. Le résultat de cette détection automatique est par exemple une surimpression lumineuse sur le tronçon de vaisseau. Puis le praticien déplace des autres index, des segments, le long et en travers de l'axe du tronçon, au besoin avec la boule ou avec un potentiomètre, et valide la position d'un serpent d'un côté de la sténose. Ce segment sert de référence pour mesurer les caractéristiques du vaisseau à un endroit normal de ce vaisseau. Enfin le praticien sélectionne un autre segment qu'il vient mettre en place au droit de la sténose. Un processeur numérique calcule alors automatiquement, et affiche dans l'écran, le diamètre sain, le diamètre sténosé et le pourcentage de rétrécissement. La quantification de la sténose peut être géométrique ou en densité.

Dans l'invention, on remédie à cette complexité de manoeuvre, en limitant d'une part le nombre des commandes, et en rendant d'autre part chacune d'elles plus complète.

Dans l'invention, le praticien peut n'avoir à agir que sur la boule de déplacement et sur la fonction d'agrandissement. Il n'y a plus de choix d'index ou de segments. La fonction d'agrandissement, en permettant une représentation agrandie de l'image d'une partie seulement de cette image, conduit à limiter automatiquement en une opération simple le domaine sur lequel le traitement automatique doit être effectué. Par manoeuvre de la boule de déplacement, il est alors possible de sélectionner dans l'image quelle partie agrandie on cherche à montrer. En quelque sorte l'écran sert de cadre au domaine d'étude. Lorsque la partie utile des vaisseaux a été placée dans ce cadre, en étant agrandie de manière à ce qu'on ne voit plus qu'elle, on lance en une unique opération, une recherche automatique des caractéristiques de la sténose.

Compte tenu du faible nombre d'ordres qu'il faut donner, il est alors possible d'utiliser une commande vocale en remplacement de la boule ou de la souris. Cette commande vocale peut n'avoir que sept ordres : elle est simple. Cette commande vocale peut aussi être remplacée par une télécommande, à infrarouge par exemple. Dans ce cas aussi le praticien peut rester à proximité du patient. Quatre ordres concernent le déplacement : "en haut, en bas, à droite, à gauche". Deux ordres concernent l'agrandissement : "plus ou moins agrandi". Un dernier ordre concerne l'opération de mesure automatique : "mesure". La mise en oeuvre de la commande vocale dans une salle de radiologie ne présente aucun danger, ni pour le patient, ni pour le système puisqu'elle ne concerne que des procédés de calcul numérique. L'interactivité de l'invention permet alors au praticien d'effectuer une quantification à mains libres, tout en intervenant chirurgicalement sur le patient. Cette ergonomie de fonctionnement n'est rendue possible que par la simplification, et l'augmentation corrélative de la puissance, des commandes de l'invention.

L'invention a donc pour objet un procédé de détermination des caractéristiques d'une structure vasculaire particulière, notamment d'une sténose dans le but de la soumettre à une angioplastie transluminale, comportant les étapes suivantes,

    – on affiche sur un écran de visualisation une image vasculaire concernée par la structure particulière à étudier, cette image étant constituée d'un ensemble de points d'image, ou pixels, chaque pixel de l'image correspondant, dans une mémoire d'image en relation avec l'écran, à une cellule mémoire de cette mémoire d'image, cette cellule correspondante étant chargée d'une valeur de luminosité et/ou de couleur du pixel dans l'image, caractérisé en ce qu'il comporte les étapes suivantes

    – on agrandit, dans l'écran, l'image de la structure particulière et de son environnement proche,

    – on évalue automatiquement les caractéristiques relatives de la structure particulière en fonction des caractéristiques des parties des vaisseaux contenues dans cet agrandissement de l'image.

L'invention sera mieux comprise à la lecture de la description qui suit et à l'examen des figures qui l'accompagnent. Celles-ci ne sont données qu'à titre indicatif et nullement limitatif de l'invention.

Les figures montrent :

    – figure 1: un dispositif utilisable pour la mise en oeuvre du procédé de l'invention ;

    – figures 2a et 2b : des représentation d'étapes du principe du calcul automatique des caractéristiques de la sténose ;

    – figures 3a et 3b : des représentations des signaux traités dans les étapes précédentes ;

    – figure 4 : un procédé de lissage permettant, en utilisant l'agrandissement, de présenter des images adoucies.

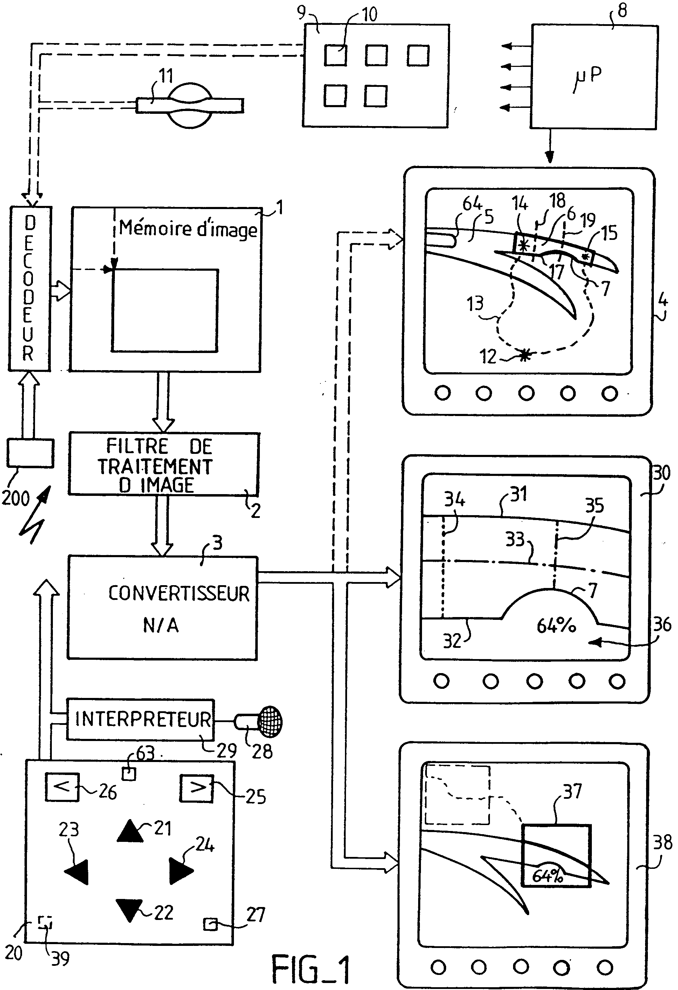

La figure 1 montre un dispositif utilisable pour la mise en oeuvre du procédé selon l'invention. Ce dispositif fait usage d'une image numérisée enregistrée dans une mémoire d'image 1. L'image numérisée est acquise normalement en temps réel, alors qu'un patient est allongé dans un appareil de radiologie non représenté. Ce patient est soumis à une irradiation X et les rayons X qui le traversent sont détectés, par exemple au moyen d'un écran intensificateur d'images radiblogiques couplé à une caméra. Les résultats de ces détections sont numérisés et envoyés, selon des procédés connus dans la mémoire d'image 1. La mémoire d'image est en relation, en particulier par l'intermédiaire d'un filtre de traitement d'image 2 et d'un convertisseur numérique analogique 3, avec un moniteur de visualisation 4. Le moniteur 4 montre, selon l'état de la technique, une image dans laquelle un vaisseau 5 présente dans une de ses branches 6 une sténose 7: un rétrécissement. La mémoire 1, le filtre 2, le convertisseur 3 et le moniteur 4 sont sous le contrôle d'un microprocesseur 8.

Dans l'état de la technique, comme dans l'invention, le praticien sélectionne dans les images qui lui ont été montrées, celle sur laquelle il décide d'effectuer la mesure. Cette image est alors stockée dans la mémoire d'image 1. Dans l'état de la technique, au moyen d'un clavier 9 muni de boutons de commande tels que 10 et d'une boule de déplacement 11 (ou d'une manière équivalente d'une souris), on provoque la sélection d'index tels que 12 et leur déplacement 13 en amont 14 ou en aval 15 de la sténose. Lorsque la sténose a été ainsi encadrée, avec un autre bouton de commande, on détecte alors automatiquement, par un procédé de traitement d'image programmé dans le microprocesseur, le contour 17 du vaisseau dans la région concernée. Puis on déplace encore une fois avec la boule 11 un autre index, par exemple un segment 18 que l'on vient placer perpendiculairement à une partie saine du vaisseau 6. Enfin, on vient placer un autre segment 19 perpendiculairement à l'endroit de la sténose, et on provoque la mesure relative.

Dans l'invention, on va utiliser des moyens similaires mais d'une manière différente. Notamment on va simplifier l'opération qui consiste à placer les index 14 et 15 de part et d'autre de la sténose. Dans ce but, on pourra utiliser un clavier spécial 20 muni de quatre touches de direction 21 à 24, et de deux touches d'agrandissement 25 de rapetissement 26, de l'image. Le boîtier de commande 20 comporte également une autre commande 27 par laquelle on ordonne la mise en oeuvre automatique de la mesure. Le boîtier de commande 20 peut être remplacé par l'association d'un microphone 28 et d'un interpréteur 29 de commande vocale. Un tel interpréteur est par exemple le système dit DATAVOX de la société VECSYS, France. On peut aussi utiliser les systèmes réalisés par les sociétés VOTAN et VERBEX, U.S.A. Au besoin le boîtier de commande 20 peut être un boîtier de télécommande en relation avec un récepteur de télécommande 200 lié au système.

Dans ces conditions le praticien peut rester à proximité du patient allongé dans la machine de radiologie. Il n'a pas à se déplacer vers une console de commande éloignée dans la salle de radiologie. En effet, les salles de radiologies modernes sont munies de différents appareils suffisamment espacés les uns des autres pour que toutes leurs manoeuvres puissent être autorisées sans se gêner mutuellement. Le système central de gestion de tout ces appareils est donc forcément éloigné de chacun d'eux. Ceci justifie l'utilisation d'une télécommande. Encore faut-il que cette utilisation soit plausible comme c'est le cas avec le système simple de l'invention.

L'idée de l'invention est la suivante. Alors que le praticien voit sur son moniteur l'image telle qu'elle est représentée sur le moniteur 4, il demande avec la touche d'agrandissement 25, ou avec un ordre équivalent reçu par le micro 28, l'agrandissement de l'image qui lui est présentée. Par exemple, il commande l'ordre "zoom plus". Puis de manière à faire apparaître dans l'écran la partie sténosé du vaisseau qui l'intéresse, il actionne les flèches de direction 21 à 23 ou envoie les ordres "en haut, en bas, à gauche, à droite" respectivement correspondant, de manière à ce que l'image de la sténose 7 vienne se présenter sensiblement au milieu de l'écran du moniteur. Ceci est représenté sur le moniteur 30. Ayant agit de cette manière, le praticien a fait plus que de restreindre le champ d'analyse, il en a provoqué une délimitation adéquate.

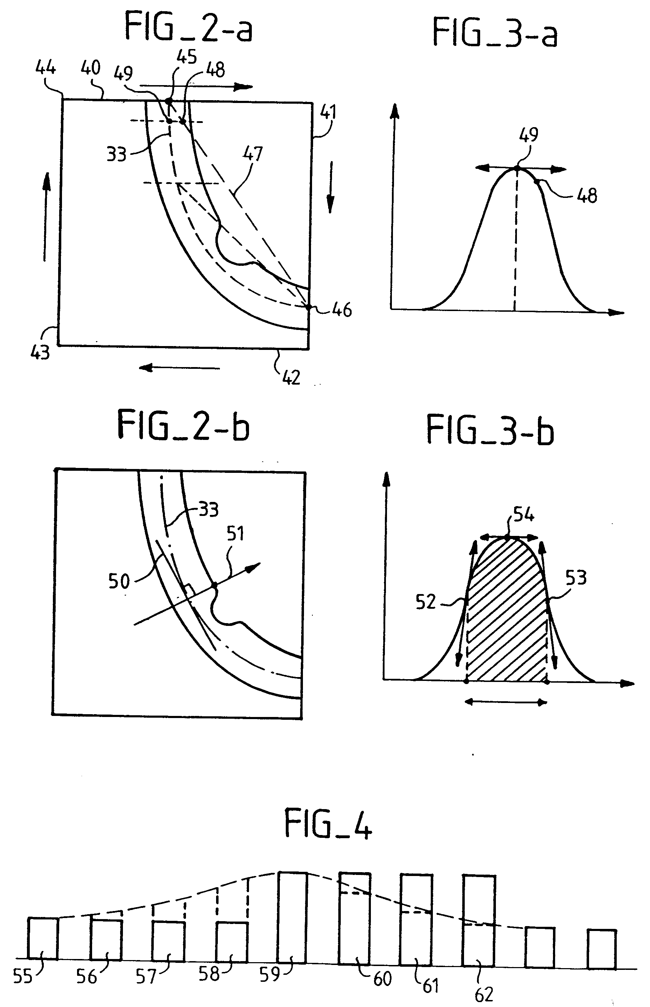

Quand il lance l'ordre de mesure, ou lorsqu'il appuie sur la commande 27, ce praticien provoque alors l'exécution des opérations suivantes. Ces opérations seront par ailleurs expliquées en regard des figures 2a et 2b. Premièrement les contours 31 et 32 du vaisseau dans l'image agrandie sont contrastés plus fortement. L'axe moyen 33 du vaisseau est ensuite déterminé par rapport aux bords 31 ou 32 comme étant l'endroit dans ce vaisseau, où le contraste radiologique est le plus fort. Par rapport à cet axe 33, on calcule ensuite automatiquement l'orientation de segments tels que 34 et 35 perpendiculaires à l'axe moyen 33. Puis, on recherche quel est le segment 34, délimité par les bords 32 et 33, qui est le plus grand. Et on cherche ensuite le segment 35 qui dans les mêmes conditions est le plus petit. Le segment 35 se trouve normalement placé à l'endroit de la sténose. On affiche ensuite un résultat 36, à l'endroit du segment 35 et de la sténose, le pourcentage de rétrécissement que celle-ci représente.

Il est important de noter que ce système de recherche automatique se substitue au déplacement et à l'orientation des index 18 et 19 dans l'état de la technique citée. Comme le traitement est automatique, il ne nécessite pas d'intervention de la part du praticien. La commande "MESURE" est donc à ce niveau suffisamment puissante pour que son exécution ne mobilise pas l'attention de ce praticien. On peut aussi noter qu'il y a plusieurs façons de mesurer le pourcentage de rétrécissement. Dans une première façon, ce pourcentage de rétrécissement est exprimé géométriquement. On mesure dans ce cas, en termes de pixel, les longueurs respectives des segments 34 et 35 sur l'écran. Dans une deuxième façon, qui est dite mesure en densité, on mesure l'intégrale du signal de contraste radiologique sur le segment 34 d'une part et sur le segment 35 d'autre part. On affiche les pourcentages respectifs. Les praticiens peuvent être habitués à l'une ou l'autre mesure auquel cas l'ordre "MESURE" pourra être remplacé par un couple d'ordres : "GEOMETRIQUE" ou "DENSITOMETRIQUE".

L'utilisation de l'agrandissement va de pair avec le fait que le moniteur sur lequel sont affichés les résultats des calculs est normalement un moniteur situé à proximité de la console générale de commande et n'est pas le moniteur de visualisation en direct de la radioscopie. Ce dernier se trouve à proximité du lit sur lequel est couché le patient et près duquel opère le praticien. Le fait de montrer une image agrandie permet donc à ce praticien, qui peut se trouver assez éloigné du moniteur de mesure, de bien voir ce qui se passe.

En variante, on peut remplacer la boîte de mesure constituée par l'écran lui même par une boîte de mesure 37. Cette boîte 37 est représentée sur le moniteur 38. Ce moniteur 38 est normalement aussi celui qui se trouve près de la console de commande. La boîte de mesure 37 est essentiellement constituée par un motif, un dessin, à l'intérieur duquel on va placer la sténose. Cette boîte de mesure 37 est concrétisée par un contour surbrillant ou coloré. Elle peut être affichée sur le moniteur 38 par envoi d'une commande supplémentaire ou par action sur un bouton de commande supplémentaire 39 du boîtier 20. Dans ce cas, par cette action la boîte apparaît sur l'écran, par exemple au début dans le coin supérieur gauche de ce dernier. Elle est déplacée, au moyen des commandes 21 à 24, jusqu'à l'emplacement adéquat autour de la sténose. Eventuellement on peut aussi utiliser les commandes d'agrandissement ou de rapetissement pour provoquer l'agrandissement ou le rapetissement simultanés ou non de la boîte de mesure 37 et de la partie du réseau vascularisé proche de cette boîte montré sur l'écran 38. La forme de la boîte de mesure n'est à priori pas fixée. Cependant, pour des raisons que l'on expliquera par ailleurs, il peut être préféré qu'elle ait une forme rectangulaire ou carrée. Une fois que la mesure est lancée, il se passe la même chose avec le dessin 37 que si ce dessin représentait les bords du moniteur 30.

De préférence l'agrandissement, et le cas échéant le rapetissement seront effectués avec des multiples de 2. Dans ce cas, au lieu de visualiser toute une page mémoire de la mémoire d'image 1, on n'en visualisera que des quarts. Ceci est obtenu simplement, au moment de la lecture de la mémoire d'image en lisant, pour chaque ligne de l'image, deux fois chaque cellule de cette mémoire, qui normalement, correspondent à un pixel de l'écran 4. Comme on lit chacun deux fois, on ne peut donc en lire que la moitié. De manière à ce que l'image ne soit pas distordue en hauteur, on lit également deux fois chaque ligne de la mémoire d'image de façon à produire deux lignes sur l'écran du moniteur 4.

Compte tenu de ce que, en balayage vidéo télévision, la lecture des lignes est entrelacée, ceci revient à lire pour la deuxième demi-image, exactement la même partie de la mémoire d'image, et de la même façon, et à l'attribuer à des lignes décalées d'un cran vers le bas dans l'écran.

Comme les indices de luminosité sont stockés dans les cellules de la mémoire 1 sous une forme numérique on utilise un convertisseur numérique analogique 3 pour transformer le signal vidéo numérisé en signal vidéo analogique applicable à un moniteur classique. Cette manière de faire conduit à appliquer la valeur de luminosité stockée dans une cellule à quatre pixels différents et contigus. D'une manière connue et expliquée plus loin sur la figure 3, on peut adoucir les phénomènes de transition en des blocs de quatre pixels (ou même de seize pixels s'il y a eu un agrandissement double deux fois). Dans ce but on interpole, pour les pixels intermédiaires, les valeurs de luminosité entre celles du pixel de départ et du pixel d'arrivée. Cette interpolation est faite dans un filtre de traitement d'image 2. Dans le cas où on utilise la variante de l'écran 38, et où on n'utilise pas l'agrandissement, ces fonctionnalités sont inutiles.

Pour déplacer avec une boule, le boîtier de commande 20, ou le dispositif de commande vocale 28-29, la boîte de mesure ou la partie d'image visualisée dans l'écran, on provoque un décalage à droite, à gauche, en haut ou en bas de l'adresse de la cellule mémoire qui correspond au pixel qui sert de point de départ pour le balayage d'image. Les commandes agissent donc sur le décodeur d'accès à la mémoire 1. Elles ajoutent ou retranchent, à une adresse stockée dans un pointeur, des quantités dépendant de l'importance de l'ordre. Par exemple, en commande vocale la réitération consécutivement d'un ordre peut entraîner, la deuxième fois, un plus grand déplacement qu'un déplacement élémentaire provoqué par le premier ordre. Autrement à chaque ordre un même déplacement peut être provoqué.

La figure 2a montre comment est repéré l'axe moyen 33 d'un vaisseau circonscrit dans l'écran 30, ou par le motif 37. Un des principes de l'invention est de provoquer l'existence d'un contour 40 à 43 qui circonscrit l'image à étudier. Dans le cas normal ce contour est constitué par les bords de l'écran eux mêmes. Dans le cas de la variante il est constitué par les bords du motif 37. Ce contour, dans la mesure où il est au moins rectangulaire, ou carré, correspond à des lignes ou des rangées, et des colonnes de cellules mémoires dans la mémoire d'image. En utilisant l'adresse d'une cellule mémoire 44 située dans un angle de ce contour pour commencer, il est ensuite possible d'aller lire les valeurs de luminosité stockées dans toutes les cellules mémoires qui correspondent aux pixels du reste du contour 40 à 43 dans les lignes et les colonnes correspondantes. On peut noter sur chaque segment du contour l'adresse de la cellule qui contient le maximum de la valeur de luminosité. On trouve ainsi l'adresse de la cellule qui correspond au pixel 45 sur le segment 40, et l'adresse de la cellule qui correspond au pixel 46 sur le segment 41. On peut alors calculer les coordonnées dans l'écran, et respectivement les adresses correspondantes dans la mémoire de tous les pixels de cet écran qui se situent sur une droite 47 reliant les pixels 45 et 46. Sur cette droite 47, à une ligne de lecture dans la mémoire d'image qui correspond à une ligne suivante à celle représentée par le segment 40, on repère ainsi un pixel 48 au point d'intersection. De part et d'autre du pixel 48, dans un sens de lecture qui correspond au mode de balayage ligne, on recherche quelle est la cellule dans laquelle la valeur de luminosité stockée est la plus forte. Ceci est représenté schématiquement sur la figure 3a où l'axe vertical montre les valeurs de luminosité stockées et où l'axe horizontal marque le sens du balayage ligne. On constate que le pixel 48 n'est pas le meilleur mais qu'au contraire le pixel 49 situé plus à gauche présente le maximum d'opacité. On retient alors le pixel 49 comme autre extrémité de la droite 47. Celle-ci se transforme alors en une droite passant maintenant par les pixels 46 et 49. Ainsi de suite, on cherche à savoir si le pixel candidat (48) dans les lignes suivantes est le bon ou s'il doit être remplacé par un autre pixel (49). La collection des pixels retenus 45, 49 jusque 46 constitue l'axe moyen du vaisseau. La détermination de la pente de la droite 47, ou de ses modifications, au fur à mesure s'appelle un procédé de programmation dynamique. Dans la pratique, seule cette pente est calculée pour déterminer l'adresse du futur (48) pixel candidat dans la ligne suivante.

Une fois que l'axe moyen 33 à été déterminé, on passe à la phase suivante. Celle-ci est écrite figure 2b. dans cette phrase on mesure la largeur géométrique, ou la largeur en densité, du vaisseau au droit de tous les points de cet axe moyen. Dans ce but, connaissant l'axe moyen 33 on peut en connaître, en tous ses points, la tangente 50 ainsi que la flèche 51. Les calculs de la tangente 50 et de la flèche 51 sont des opérations connues de l'homme de l'art.

Le long de la flèche 51 il est alors possible, figure 3b, de connaître la variation de la luminosité en chaque pixel en fonction de l'abscisse le long de cette flèche. La luminosité des pixels est montrée verticalement, l'abscisse est montrée horizontalement. On peut retenir comme abscisses de pixels situés au bord du vaisseau, celles pour lesquelles la courbe de la variation de luminosité subit une inflexion 52 et 53, de part et d'autre d'un maximum 54. Lorsque l'on affiche la largeur géométrique on mesure la différence entre les abscisses des pixels 52 et 53 sur la flèche 51. Lorsqu'on affiche la densité, on calcule l'intégrale de la luminosité comprise entre ces deux abscisses. Cette dernière représente la surface hachurée sur la figure 3b. Retenir la plus petite ou la plus grande largeur revient à retenir les résultats de ces calculs selon le mode déterminé à l'avance, géométrique ou densitométrique.

La figure 4 montre le travail effectué par le filtre de traitement d'image 2. Dans le cas où il y a eu un double agrandissement, dans chaque ligne de l'image il y a des blocs de quatre pixels auxquels on doit normalement attribuer, du fait de la relecture de la mémoire d'image, des mêmes valeurs de luminosité. Par exemple, les quatre pixels 55 à 58 sont suivis des quatre pixels 59 à 62. Pour éviter l'effet de bloc qui serait ainsi provoqué, on peut accepter d'effectuer sur les pixels 56,57 et 58 une interpolation entre les valeurs des pixels 55 et 59. Dans ce but, le filtre de traitement d'image 2 comporte des moyens pour stocker les valeurs de luminosités des pixels 56, 57 et 58 tant qu'il ne connaît pas la valeur à attribuer au pixel 59. Dès que cette valeur est connue, au vol, on attribue au pixel 56 la valeur du pixel 55 plus le quart de la différence entre celle du pixel 59 et du pixel 55. Ainsi de suite pour les pixels 57 et 58.

Après avoir effectué la mesure relative de la sténose, il convient d'en faire une mesure absolue. Par une commande vocale supplémentaire "CALIBRATION", ou par un bouton de commande 63 affecté à cet effet sur le boîtier de commande 20, le praticien provoque une même suite d'opérations pour placer la boîte de mesure à l'endroit où le cathéter a été introduit dans le vaisseau du patient plutôt qu'à l'endroit de la sténose. Un tel cathéter 64 est par exemple visible sur l'écran 4. Avec la boîte de mesure le praticien vient donc se placer à l'endroit de ce cathéter. De préférence, on peut s'arranger pour que, dans cette phase de calibration, les contraintes d'agrandissement soient les mêmes que celles ayant servi à déterminer les dimensions de la sténose. Cependant, ceci n'est pas une obligation, et il pourrait suffire de tenir compte de la différence d'agrandissement d'une situation à l'autre. Le même type de traitement que celui évoqué jusqu'à présent se substitue naturellement aux recherches de contour du vaisseau. La phrase finale du traitement est seulement remplacée par une évaluation relative de la largeur de la sténose et de la largeur du cathéter.

Comme on a indiqué précédemment, on a pu préférer comme dimension normale du vaisseau la dimension de ce vaisseau qui apparaît la plus grande dans le boîtier de mesure. On connaît donc cette dimension en terme de pixels sur l'écran. Au moment de la calibration, on cherche à connaître de la même façon la largeur moyenne du cathéter, et on compare cette dimension à celle du vaisseau, de préférence l'endroit où le vaisseau est le plus rétréci. Connaissant par ailleurs la dimension du cathéter, il est alors possible au praticien d'en déduire la dimension du ballonnet à introduire pour effectuer l'angioplastie. Le praticien opère alors en deux temps. Dans un premier temps il mesure la sténose. La largeur de celle-ci le conduit ou non à pratiquer une angioplastie. Dans le premier cas le praticien effectue dans un deuxième temps la mesure de calibration.

L'invention est applicable à d'autres quantifications que celles qui concernent les sténoses. Par exemple on peut calculer aussi des volumes cardiaques. Dans ce cas, les contours, les bords de l'écran sont placés autour de ces volumes. Un jeu de médianes ou de diagonales, ou les deux, de la boîte de mesure peut permettre de repérer les limites de ces volumes. Dans ce but, on mesure la variation des luminosités affectées aux pixels situés sur ces médianes et diagonales. On relie les pixels, qui correspondent aux inflexions de ces mesures, par des segments. Et on mesure la surface contenue dans ces segments. Elle rend compte du volume.

## Revendications

1. Procédé de détermination des caractéristiques d'une structure vasculaire particulière dans une image, notamment d'une sténose (7) dans le but de la soumettre à une angioplastie transluminale, comportant les étapes suivantes,
   – on affiche sur un écran de visualisation (30) une image vasculaire totale concernée par la structure particulière à étudier et par l'environnement de cette structure particulière, cette image étant constituée d'un ensemble de points d'image, ou pixels, chaque pixel de l'image correspondant, dans une mémoire d'image (1) en relation (2,3) avec l'écran, à une cellule mémoire de cette mémoire d'image, cette cellule correspondante étant chargée d'une valeur de luminosité et/ou de couleur du pixel dans l'image,
   caractérisé en ce qu'il comporte les étapes suivantes
   – on commence par agrandir (25), l'image totale puis on déplace sur l'écran (21-24), l'image visible de la partie agrandie de l'image totale pour faire apparaître l'image de la structure particulière, et
   – on évalue entièrement automatiquement (27) les caractéristiques relatives de la structure particulière en fonction des caractéristiques des parties vasculaires vaisseaux contenues dans cet agrandissement de l'image.

2. Procédé selon la revendication 1, caractérisé en ce qu'il comporte, pour évaluer automatiquement les caractéristiques, les étapes suivantes :

– on teste les valeurs chargées dans les cellules de la mémoire d'image qui correspondent aux cotés du contour, respectivement de l'agrandissement, ou du motif, pour savoir lesquelles de ces cellules sont représentatives de milieux des vaisseaux concernés,

– on recherche par programmation dynamique à partir de ces cellules testées les cellules de la mémoire d'image qui correspondent aux milieux de ces vaisseaux,

– on détermine pour chaque cellule recherchée une orientation perpendiculaire au vaisseau,

– on recherche les cellules de la mémoire d'image qui correspondent, dans ces orientations perpendiculaires, aux bords des vaisseaux, et

– on mesure le long des orientations perpendiculaires une largeur des vaisseaux à un endroit normal d'une part et à l'endroit de la structure particulière ou sténose d'autre part, et on affiche la largeur relative,

– la sténose étant supposée placée là où cette largeur est la moins grande.

3. Procédé selon la revendication 2, caractérisé en ce que, pour mesurer la largeur,

– on détermine quelles sont les cellules de la mémoire d'image, correspondant à l'orientation perpendiculaire, pour lesquelles la courbe des valeurs chargées subit un point d'inflexion.

4. Procédé selon l'une quelconque des revendications 1 à 3, caractérisé en ce que

– on affiche en regard de l'endroit de la sténose sur l'écran la dimension géométrique relative de cette sténose par rapport à celle mesurée à l'endroit normal.

5. Procédé selon l'une quelconque des revendications 1 à 4, caractérisé en ce que

– on affiche en regard de l'endroit de la sténose sur l'écran la densité relative à l'endroit de cette sténose par rapport à celle mesurée à l'endroit normal.

6. Procédé selon l'une quelconque des revendications 4 à 5, caractérisé en ce que

– on choisit comme endroit normal celui où la quantité mesurée est la plus grande.

7. Procédé selon l'une quelconque des revendications 1 à 6, caractérisé en ce que on modifie l'agrandissement dans un rapport deux à chaque action.

8. Procédé selon l'une quelconque des revendications 1 à 7, caractérisée en ce que

– on télécommande les étapes au moyen d'un dispositif de commande vocale.

FIG_1

EP 0 475 818 A1

FIG_2-a

FIG_3-a

FIG_2-b

FIG_3-b

FIG_4

10

EP 0 475 818 A1

**Office européen des brevets** — **RAPPORT DE RECHERCHE EUROPEENNE** — Numero de la demande

EP 91 40 2356
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-4 528 585 (T.V. BOLGER) 9 Juillet 1985<br>* abrégé *<br>* colonne 1, ligne 17 - ligne 26 *<br>* colonne 13, ligne 67 - colonne 20 * | 1-4,6,7 | G06F15/70 |
| Y | PROCEEDINGS OF THE ANNUAL INTERNATIONAL CONFERENCE OF THE IEEE ENGINEERING IN MEDICINE AND BIOLOGY SOCIETY, VOL.11:1989; SEATTLE, WASHINGTON<br>Novembre 1989, IEEE,NEW YORK (US)<br>pages 354 - 355;<br>K.C. ACHARYA ET AL.: 'Automatic edge detection and diameter calculation of coronary arteries using DSA images'<br>* page 354 * | 1-4,6,7 | |
| A | IEEE TRANSACTIONS ON ACOUSTICS,SPEECH AND SIGNAL PROCESSING.<br>vol. 36, no. 9, Septembre 1988, NEW YORK US<br>pages 1501 - 1513;<br>T.N. PAPPAS ET AL.: 'A new method for estimation of coronary artery dimensions in angiograms'<br>* page 1501 - page 1502 * | 2,3,4,6,7 | DOMAINES TECHNIQUES RECHERCHES (Int. Cl.5)<br><br>G06F |
| A | IEEE TRANSACTIONS ON MEDICAL IMAGING.<br>vol. 7, no. 3, Septembre 1988, NEW YORK US<br>pages 173 - 187;<br>K. KITAMURA ET AL.: 'Estimating the 3-D skeletons and transverse areas of coronary arteries from biplane angiograms'<br>* le document en entier * | 2,3,4,6,7 | |
| A | FR-A-2 623 642 (THOMSON-RECHERCHE) 26 Mai 1989<br><br>* abrégé *<br>* page 4, ligne 12 - ligne 30 * | 2,3,4,6,7 | |

-/--

Le présent rapport a été établi pour toutes les revendications

| Lieu de la recherche | Date d'achèvement de la recherche | Examinateur |
|---|---|---|
| LA HAYE | 05 DECEMBRE 1991 | CHATEAU J.P. |

EPO FORM 1503 03.82 (P0402)

EP 0 475 818 A1

Office européen des brevets

**RAPPORT DE RECHERCHE EUROPEENNE**

Numero de la demande

EP 91 40 2356
Page 2

# DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | PATENT ABSTRACTS OF JAPAN<br>vol. 9, no. 156 (P-368)29 Juin 1985<br>& JP-A-60 029 873 ( TOSHIBA K.K. ) 15 Février 1985<br>* abrégé * | 1 | |
| A | PROCEEDINGS OF THE INTERNATIONAL SOCIETY OF OPTICAL ENGINEERING SPIE, SANTA CLARA, 12-13 FEVRIER 1990 ; SPIE, WASHINGTON (US)<br>pages 182 - 191;<br>S.J. AGGARWAL ET AL.: 'Tracking of vessel diameter fluctuations using digital image analysis'<br>* page 183 * | 2-4 | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 05 DECEMBRE 1991 | CHATEAU J.P. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)

12